Europäisches Patentamt

European Patent Office

Office européen des brevets

(19)

(11) Publication number: 0 293 037
A1

## (12) EUROPEAN PATENT APPLICATION

(21) Application number: 88200994.7

(51) Int. Cl.4: C07C 147/10

(22) Date of filing: 18.05.88

(30) Priority: 26.05.87 US 54331

(43) Date of publication of application:
30.11.88 Bulletin 88/48

(84) Designated Contracting States:
AT BE CH DE ES FR GB GR IT LI LU NL SE

(71) Applicant: AKZO N.V.
Velperweg 76
NL-6824 BM Arnhem(NL)

(72) Inventor: Engel, Michael J.
52 Jackson Place
White Plains New York 10603(US)
Inventor: Anzovino, William T.
Rd 2, Box 452
Peekskill New York 10566(US)
Inventor: Tomko, John
200 Beacon Hill Drive
Dobbs Ferry New York 10522(US)
Inventor: Abramson, Alan
60-104 Pinewood Road
Hartsdale New York 10530(US)

(74) Representative: Sieders, René et al
AKZO N.V. Patent Department (Dept. CO)
P.O. Box 9300
NL-6800 SB Arnhem(NL)

(54) Preparation of 4,4'-dihydroxydiphenyl sulfone.

(57) A method of preparing 4,4'-dihydroxyphenyl sulfone wherein 4,4'-dihydroxydiphenyl sulfone is prepared by reaction of phenol with sulfuric acid in the presence of a suspending agent and wherein the proportion of 4,4'-dihydroxydiphenyl sulfone obtained is significantly increased in a process whereby the reaction medium comprising sulfonic acid, a suspending agent, and phenol are maintained at a high temperature for a period of time to produce 4,4'-dihydroxydiphenyl sulfone in amounts in excess of 95% and wherein recovery of the product is obtained by separation from the reaction mixture.

FIG.2

# PREPARATION OF 4,4'-DIHYDROXYDIPHENYL SULFONE

## BACKGROUND OF THE INVENTION

### Field of the Invention

The invention is directed to the preparation of 4,4'-dihydroxydiphenyl sulfone. The invention in particular relates to the preparation of 4,4'-dihydroxydiphenyl sulfone in a process wherein small amounts of the by-product 2,4'-dihydroxydiphenyl sulfone isomer are obtained and wherein purification of the product comprises a single recrystallization step.

### Related Art

Production of 4,4'-dihydroxydiphenyl sulfone by available methods generally results in the unavoidable obtaining of reaction mixtures comprising the desired 4,4'-dihydroxydiphenyl sulfone in a mixture with substantial amounts of by-product 2,4'-dihydroxydiphenyl sulfone. The 4,4' product is generally produced in yields of <95.0%. This is the case, for example, when the desired 4,4' isomer is produced from starting materials comprising phenol and concentrated sulfuric acid, or phenol and p-phenol sulfonic acid. The separation of the 4,4' isomer from admixed 2,4' isomer heretofore has presented considerable difficulties and can generally not be carried out in large scale operations by ordinary practical scale separating means. Separation can be, however, difficultly accomplished by multiple recrystallizations to obtain a high purity (99.5 +) 4,4' material.

It is known to prepare 4,4'-dihydroxydiphenyl sulfone by the reaction of phenol and sulfuric acid (the proportion of phenol being in excess of the stoichiometric proportion of 2 moles of phenol/mole of sulfuric acid), but the desired product is usually accompanied by an unacceptably high proportion of the 2,4'-dihydroxydiphenyl sulfone isomer.

Japanese Patent Publication No. 51-98239 proposes to carry out a reaction between a phenol and sulfuric acid in the presence of a solvent in which 4,4'-dihydroxydiphenyl sulfone is less soluble than the 2,4'-dihydroxydiphenyl sulfone. The solvent is gradually evaporated from the reaction system. The 4,4'-dihydroxy isomer formed separates and, since the two isomers are in equilibrium, isomerization of the 2,4'-dihydroxy isomer proceeds. This proposed method does, however, require careful control of the reaction conditions to ensure an appropriate balance between the rate of removal of the solvent and the rate of isomerization. Furthermore, the reaction product, after removal of the solvent, may be in the form of a rather intractable solid mass.

British Patent Application No. 2,030,566 discloses a process for the preparation of impure (<99.5%) 4,4'-dihydroxydiphenyl sulfone wherein the recovered yield of 4,4'-dihydroxydiphenyl sulfone is increased by heating a mixture containing 4,4'-dihydroxydiphenyl sulfone and 2,4'-dihydroxydiphenyl sulfone in the presence of an acidic catalyst and an organic solvent. The solvent utilized in the process disclosed is 1,2-dichlorobenzene and the catalysts utilized are organic acids or aqueous mineral acids.

German Patent No. 2,708,388 discloses a process for preparing 4,4'-dihydroxydiphenyl sulfone by reaction of phenol with sulfuric acid in the presence of a solvent, ortho dichlorobenzene, which was said to form an azeotrope with the water of reaction. The water of reaction was removed by azeotropic distillation, then the solvent was removed by distillation leaving an intractable mass. The yield of the product obtained by the process described was said to be 91.2% and contained less than 0.8% of the 2,4' isomer.

The presence of the by-product 2,4' isomer even in relatively small amounts in the purified product greatly restricts the field of the application of such contaminated 4,4'-dihydroxydiphenyl sulfone.

One method of separating 4,4'-dihydroxydiphenyl sulfone from the 2,4' isomer is disclosed in U.S. Patent No. 3,065,274. The method of separation disclosed is by conducting the reaction in the presence of sym-tetrachloroethane solvent at the boiling temperature of the solvent, about 140° C at the pressure employed. This sym-tetrachloroethane is combined with the mixed isomer to be separated. The mixture is then cooled to a temperature in the range of about 80° C to about 125° C and preferably about 100° C and filtered without lowering to any substantial degree the temperature, thereby obtaining a filter cake consisting essentially of 4,4'-dihydroxydiphenyl sulfone free from any substantial amounts of 2,4'-dihydroxydiphenyl sulfone, and the filtrate consisting essentially of sym-tetrachloroethane containing 2,4'-dihydroxydiphenyl sulfone in solution. This system suffers from the fact that if the system is cooled below 80°C during or prior to filtration, the 2,4' isomer precipitates out of solution. In addition, the product obtained by this process still contains several percent of the 2,4' isomer based on the melting point provided. In addition, the sym-tetrachloroethane can react to a small extent with sulfuric acid, resulting in product impurities.


## SUMMARY OF THE INVENTION

The present invention is a process for producing substituted and unsubstituted 4,4'-dihydroxydiphenyl sulfone in high yields (>95%) and wherein the desired product can be separated in a one-step process from the 2,4' by-product isomer by recrystallization to yield a product with purity in excess of 99.5%. In the process of the invention, phenol is reacted with sulfuric acid in the presence of a suspending agent and an azeotroping agent at a temperature and for a time sufficient to produce the 4,4'-dihydroxydiphenyl sulfone in yields greater than 95%. The process of the invention further comprises recovery of the 4,4'-dihydroxydiphenyl sulfone from a mixture containing the 2,4'-dihydroxydiphenyl sulfone isomer by filtering, redissolving the product, and recrystallizing.


## BRIEF DESCRIPTION OF THE DRAWINGS

Fig. 1 represents the preparation of 4,4'-dihydroxyphenyl sulfone both isothermally and allowing the temperature to rise gradually over time.

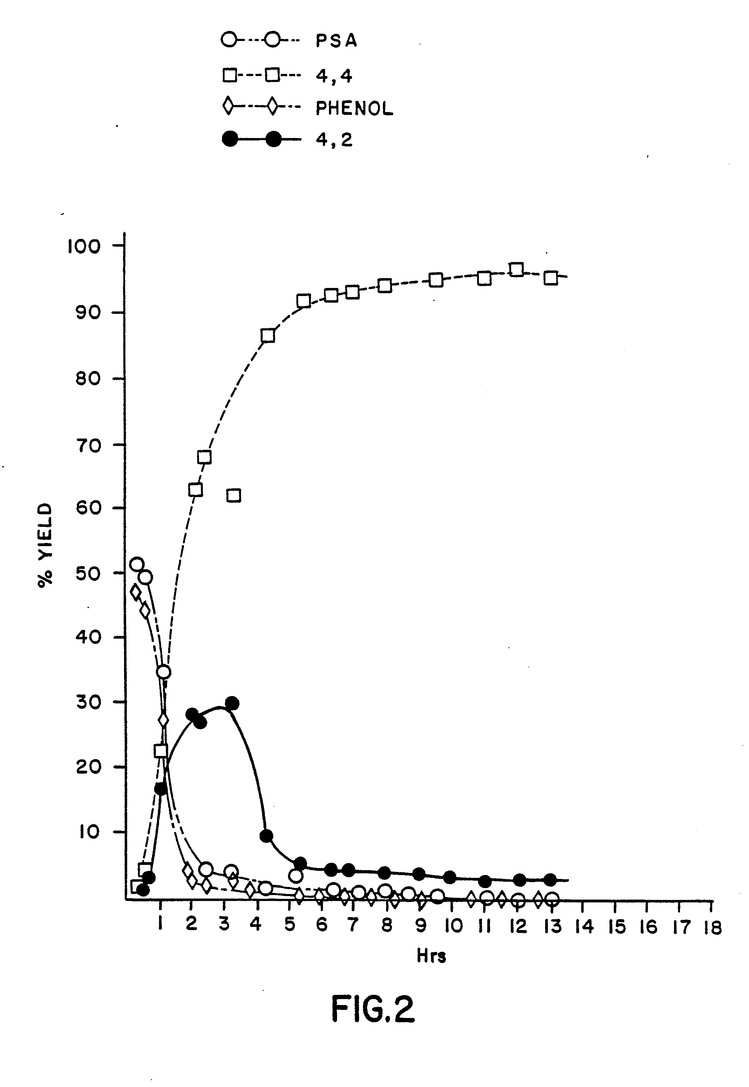

Figs. 2 and 3 show the preparation of 4,4'-dihydroxydiphenyl sulfone wherein the reaction is conducted at a temperature and time sufficient to maximize product yield.


## DETAILED DESCRIPTION OF THE INVENTION

The invention, as previously indicated, relates to the preparation of substituted and unsubstituted 4,4'-dihydroxydiphenyl sulfone by the reaction of a phenol with sulfuric acid in the presence of a suspending agent and an azeotroping agent for a time and at a temperature sufficient to maximize the product yield. The substituted product can contain any non-interfering substituents such as, for instance, alkyl or chloride groups. The invention further comprises a method of recovering the 4,4'-dihydroxydiphenyl sulfone in a purified state without undue purification steps.

The suspending agent utilized in the process of the invention is desirably one which has a boiling point above about 120°C, and which is inert under the reaction conditions.

Suitable suspending agents for practicing the invention are, therefore, straight or branched aliphatic hydrocarbons or mixtures thereof, and aliphatic halohydrocarbons and mixtures of the above which meet the above criteria. A preferred suspending agent for practicing the invention is the isoparaffin ISOPAR H® brand of hydrocarbons.

It is necessary to remove water formed during the process. An azeotroping agent is therefore utilized to remove water. Azeotroping agents such as ISOPAR E®, can be utilized. In some instances, the suspending agent may also function as an azeotroping agent. Azeotroping agents can comprise any non-reactive hydrocarbon material that boils below the reaction temperature.

The reaction is conducted at temperatures above about 150°C and preferably at temperatures of from 170°C to 200°C. The temperature of the reaction is generally dependent on the specific suspending agent and azeotroping agent utilized since it is preferred to conduct the reaction at as high a temperature as possible to maximize the rate without causing agglomeration.

Stoichiometric quantities of the reactants can be utilized in the process, although in the practice of the invention a 1-10% excess of phenol is preferred. Sufficient phenol is used to allow for the amount retained in the organic phase during the process.

Generally, in the practice of the invention, the amount of phenol to sulfuric acid should be from about 2-3 moles to about 1 mole, and preferably from about 2.2 moles to about 1 mole. The amount of the azeotroping agent used will vary depending on the reaction temperature. At higher temperatures, smaller amounts are required than at lower reaction temperatures. The amount of azeotroping agent used should be sufficient to maintain a reflux and effectively remove water.

The synthesis of the process disclosed is as follows, with Ar representing a phenyl ring:

3

$$ArOH + H_2SO_4 \xrightarrow{\text{to } \sim 120^{\circ}C-} HOArSO_3H + H_2O \qquad (1)$$

$$OHArSO_3H + ArOH \xrightarrow[\sim 220^{\circ}C]{\sim 120^{\circ}C-} HOArSO_2ArOH + H_2O \qquad (2)$$

In carrying out the overall process of the invention, phenol is heated with the suspending agent and then reacted with $H_2SO_4$ at a temperature of from about 120°C to about 200°C and generally from about 120°C to about 220°C. During the reaction, it is necessary to provide sufficient agitation to prevent particle agglomeration. Initially, the phenol and suspending agent are heated together at temperatures from about 100°C to about 120°C. After addition of the acid, the reactor is heated to a temperature above about 120°C and preferably from about 150°C to about 200°C, depending on the suspending agent and azeotroping agent used. This reaction is continued for the time necessary to substantially complete the primary conversion to 4,4'-dihydroxydiphenyl sulfone. A variation of the above is heating the acid with the phenol and suspending agent for a time below the reaction temperature to substantially form phenol sulfonic acid before increasing the temperature above 120°C. Completion of the reaction process can be determined by high pressure liquid chromatography, and by monitoring water collected in the Dean-Stark strap. An azeotroping agent is used during the reaction process to remove the water produced during the reaction.

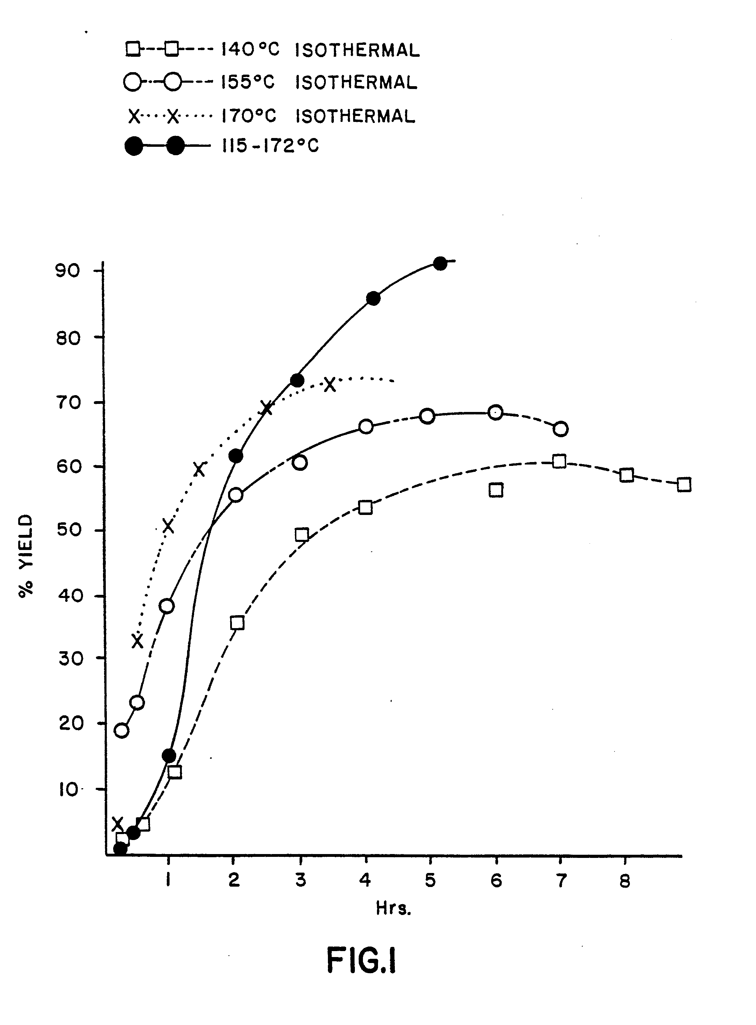

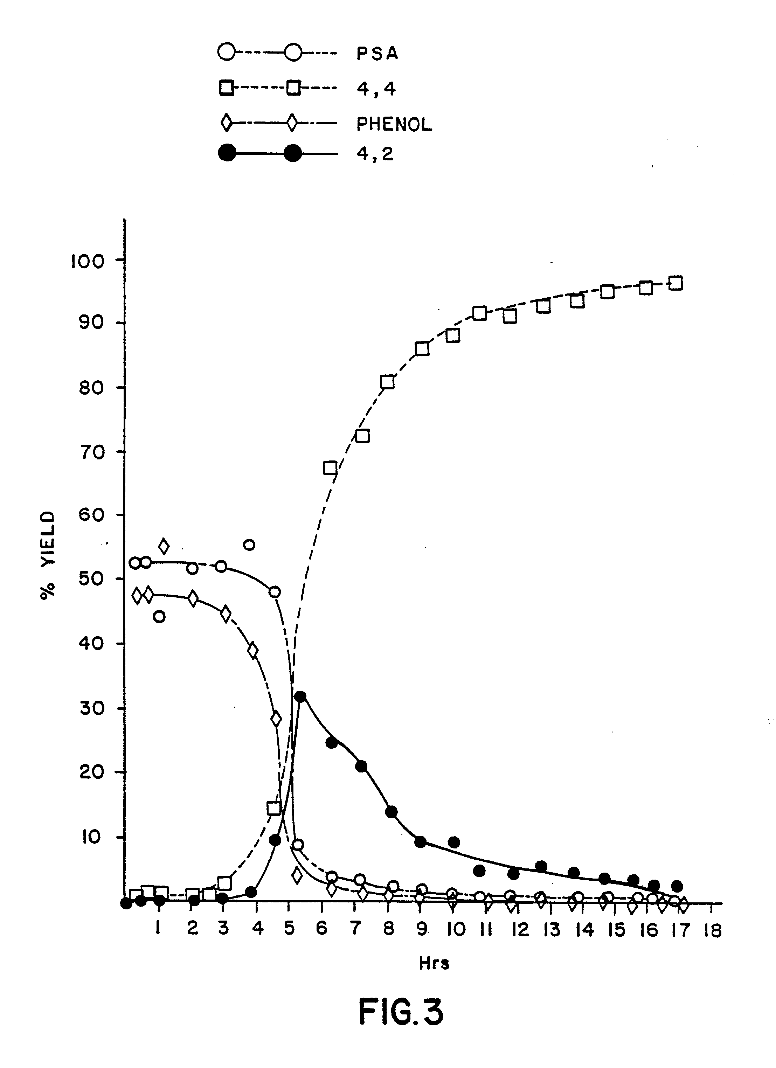

The invention can further be described by reference to the Drawings. Fig. 1. shows the production of 4,4'-dihydroxydiphenyl sulfone using isothermal methods and a process whereby the reaction is conducted over time while gradually increasing the reaction temperature from 115°C to 172°C. A yield of only 90% of the 4,4' isomer product was realized due to the premature termination of the reaction at process temperatures of 150°C to 200°C. The other results shown in Fig. 1 are indicative of the much lower yields of the product which are obtained by reacting at too low a temperature for an insufficient time. Figs. 3 and 4 show that high yields of the product 4,4' isomer can be achieved with low yields of the 2,4' isomer (3%) when the reaction is maintained at a high temperature for sufficient time to substantially complete the reaction.

The recovery of the crude product is accomplished by reducing the temperature of the reaction mixture to above about 80°C and preferably about 90°C and removing the suspending agent as by simple decantation. Water at a temperature above about 80°C is then added to dilute the reaction mixture and to solubilize unreacted phenol sulfonic acid. The reaction mixture is then filtered while still maintaining the temperature of the mixture above about 80°C. The product filter cake is then washed with the suspending agent which is also maintained at a temperature above about 80°C. The recovery of the purified product is conducted in a single recrystallization to obtain a 99.5% or higher, 4,4' dihydroxydiphenyl sulfone.

<u>EXAMPLE 1</u>

100.13 grams of phenol, 130.00 grams of ISOPAR H® suspending agent and 8.77 grams of ISOPAR E® azeotroping agent were charged to a 500 ml flask and heated to 100°C. Then 52.1 grams of sulfuric acid was added over 30 minutes and heat was added over 5 hours to bring the temperature to 170°C. Heating was continued at 172°C for another 3 hours. The results are as shown. Fig. 1 shows the results of this experiment.

| Run Time hrs/min | T °C Pot | % Sulfonic Acid | % Phenol | % 4,4'- isomer | % 2,4' isomer |
|---|---|---|---|---|---|
| 0/30 | 111 | 55 | 44.2 | 0.5 | 0.2 |
| 1/00 | 149 | 48.5 | 44.6 | 3.9 | 2.9 |
| 1/45 | 157 | 38.5 | 33.8 | 14.7 | 12.1 |
| 2/45 | 165 | 5.1 | 3.0 | 61.3 | 28.3 |
| 3/35 | 170 | 3.0 | 1.4 | 72.0 | 23.1 |
| 4/45 | 172 | 1.8 | 1.4 | 85.6 | 10.4 |
| 6/00 | 172 | 0.9 | 1.6 | 90.1 | 6.6 |

Note: $H_2SO_4$ all added at time 0.

EXAMPLE 2

100.0 gms of phenol (1.06 mole), 139 cc of ISOPAR H®, and 5 cc of ISOPAR E® were placed into a 250 cc 3-neck reaction flask (under $N_2$ blanket) fitted with a thermometer, dropping funnel, mechanical stirrer, oil-heat bath, and a sampling port and a Dean-Stark trap.

The reaction mixture was then heated to 100°C and dropwise was added 52.1 gms of concentrated sulfuric acid (0.53 mole) while stirring over a period of 25 min.

Once the $H_2SO_4$ addition was completed, the oil-bath temperature was gradually raised until the pot temperature reached 170°C (7 hours). The reaction mixture temperature was kept at 170°C for an additional 12 hours.

Completion of the reaction was determined by collection of water in the Dean-Stark trap and by high pressure liquid chromatography.

| Run Time hrs/min | T °C Pot | ml $H_2O$ Trap | % Sulfonic Acid | % Phenol | % 4,4'- isomer | % 2,4' isomer |
|---|---|---|---|---|---|---|
| 0/15 | 97 | 0 | 52.4 | 47.1 | 0.5 | 0 |
| 2/00 | 100 | 0 | 52.3 | 47.3 | 0.4 | 0 |
| 3/00 | 100 | 0 | 54.5 | 44.9 | 0.6 | 0 |
| 3/45 | 130 | 0 | 55.3 | 39.4 | 3.1 | 1.8 |
| 4/55 | 150 | 0.2 | 45.4 | 29.3 | 14.8 | 10.0 |
| 5/15 | 161 | 3.0 | 8.8 | 4.8 | 54.4 | 32.0 |
| 6/20 | 165 | 6.8 | 3.8 | 2.3 | 68.0 | 24.8 |
| 7/10 | 170 | 8.6 | 3.0 | 1.4 | 73.5 | 20.6 |
| 8/25 | 170 | 15.7 | 1.8 | 1.7 | 81.3 | 13.7 |
| 9/25 | 170 | 16.2 | 1.5 | 1.2 | 86.1 | 9.7 |
| 10/25 | 170 | 16.2 | 1.0 | 1.6 | 87.0 | 9.5 |
| 13/40 | 170 | 16.7 | 0.7 | 1.3 | 91.6 | 5.7 |
| 16/50 | 171 | 16.7 | 0.6 | trace | 96.1 | 2.6 |

Note: At % time all $H_2SO_4$ addition completed.

5

## EXAMPLE 3

100.0 gms of phenol, 139 cc of ISOPAR H®, 8.7 cc ISOPAR E® were placed into a 250 cc 3-neck flask (equipped as in Example 2).

Sulfuric acid (52.1 gms) was added dropwise at 100°C in 30 min.

The reaction mixture was then heated up to 170°C in 2 hours, then kept at 170°C for additional 12 hours. All the sulfuric acid was added prior to commencing the run time.

| Run Time hrs/min | T °C Pot | ml H$_2$O Trap | % Sulfonic Acid | % Phenol | % 4,4'- isomer | % 2,4' isomer |
|---|---|---|---|---|---|---|
| 0/5 | 106 | 0 | | | | |
| 0/15 | 130 | 0 | 51.2 | 47.0 | 1.0 | 0.4 |
| 0/30 | 148 | 0 | 49.8 | 44.5 | 3.7 | 2.0 |
| 1/00 | 157 | 2.9 | 34.9 | 26.7 | 22.5 | 16.0 |
| 2/00 | 168 | 13.0 | 4.1 | 2.4 | 63.2 | 27.9 |
| 3/20 | 170 | 15.0 | 3.9 | 3.5 | 61.3 | 30.0 |
| 4/50 | 170 | 16.1 | 1.5 | 1.5 | 86.7 | 9.4 |
| 5/50 | 170 | 17.8 | 3.6 | <1.0 | 92.0 | 4.4 |
| 7/55 | 170 | 17.8 | 0.4 | 1.3 | 94.2 | 3.8 |
| 15/20 | 170 | 17.8 | 0.1 | 1.0 | 95.6 | 2.9 |
| 16/20 | 170 | 17.8 | 0.1 | 0.9 | 95.6 | 3.0 |

## Claims

1. A process for forming unsubstituted 4,4'-dihydroxydiphenyl sulfone in above about 95% purity by reaction of phenol with sulfuric acid which comprises reacting the phenol and sulfuric acid in the presence of a suspending agent and an azeotroping agent at temperature conditions and for a time sufficient to produce the 4,4'-dihydroxydiphenyl sulfone in said purity without a recrystallization step.

2. The process of Claim 1 wherein the suspending agent is a straight chained aliphatic hydrocarbon.

3. The process of Claim 1 wherein the suspending agent is a branched chain aliphatic hydrocarbon.

4. The process of Claim 3 wherein the suspending agent is an isoparaffinic hydrocarbon.

5. The process of Claim 1 wherein the azeotroping agent is an isoparaffinic hydrocarbon.

6. The process of Claim 1 wherein the phenol is heated with the suspending agent and the azeotroping agent to a temperature of from about 100°C to about 120°C before the addition of the sulfuric acid.

7. The process of Claim 1 wherein the reaction mixture, after the addition of the sulfuric acid, is heated to a temperature of from about 150°C to about 220°C for a time sufficient to substantially produce the 4,4'-dihydroxydiphenyl sulfone product.

8. A process for producing 4,4'-dihydroxydiphenyl sulfone of the formula

$HOC_6H_4SO_2C_6H_4OH$

in above about 95% purity without a recrystallization step comprising the steps of:

(a) heating a phenol, suspending agent, and an azeotroping agent to a temperature of from about 100°C to about 120°C to form a heated mixture;

(b) adding to the heated material sufficient sulfuric acid to produce a reaction mixture;

(c) heating the reaction mixture to a temperature of from about 150°C to about 220°C to produce the sulfone; and

(d) separating and recovering the sulfone from the reaction mixture.

9. The process of Claim 8 wherein the suspending agent is a straight chained aliphatic hydrocarbon.

10. The process of Claim 9 wherein the suspending agent is a branched chain aliphatic hydrocarbon.

11. The process of Claim 10 wherein the suspending agent is an isoparaffinic hydrocarbon.

12. The process of Claim 8 wherein the azeotroping agent is an isoparaffinic hydrocarbon.

13. The process of Claim 8 wherein the phenol is heated with the suspending agent and the azeotroping agent to a temperature of from about 100° C to about 120° C before the addition of the sulfuric acid.

14. The process of Claim 8 wherein the reaction mixture, after the addition of the sulfuric acid, is heated to a temperature of from about 150° C to about 220° C for a time sufficient to substantially produce the 4,4'-dihydroxydiphenyl sulfone product.

15. The process of Claim 8 wherein the crude product, after completion of the process, is recovered by decantation from the suspending agent and washed with water and suspending agent.

FIG.I

FIG.2

FIG.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-3 318 956 (M.L. MAUSNER) ----- | | C 07 C 147/10 |
| | | | **TECHNICAL FIELDS SEARCHED (Int. Cl.4)** |
| | | | C 07 C 147/00 |

The present search report has been drawn up for all claims

| Place of search | Date of completion of the search | Examiner |
|---|---|---|
| THE HAGUE | 29-08-1988 | VAN GEYT J.J.A. |